# EUROPEAN PATENT APPLICATION

(11) **EP 1 779 891 A1**
(43) Date of publication of application: **02.05.2007**
(21) Application number: 05077475.1
(22) Date of filing: 28.10.2005
(51) Int. Cl.: A61N 5/06, A61K 41/00

(54) **Method of activating a photosensitizer**

(71) Applicant: Kurkayev, Abdula, Angyal Utca 13 apt. 2 1094 Budapest (HU)
(72) Inventor: Kurkayev, Abdula, Angyal Utca 13 apt. 2 1094 Budapest (HU)
(74) Representative: Winckels, Johannes Hubertus F.

(57) **Abstract**

The invention relates to a method of activating a photosensitizer, wherein for the photosensitizer nanoparticles of a catalyst capable of catalyzing the production of active oxygen (1) is selected, which is further subjected to irradiation by light (5). Preferably, heterocrystal minerals are used as a source of nanoparticles. Preferably, the photosensitizer is combined with a liquid (3), to which a suitable amount of oxygen gas (4) is added. Still preferably, the photosensitizer is chemically coupled to a DNA-molecule and a suitable anti-metabolic agent. The invention further relates to a method for treating a health disorder using activated photosensitizer provided in nanoparticle form, whereby the activated nanoparticle photosensitizer is administered to a recipient (7).

## Description

The invention relates to a method of activating a photosensitizer.

Photosensitizers relate to classes of materials catalyzing a production of active oxygen forms in an oxygenated medium, notably singlet oxygen, when subjected to light irradiation. Usually, pigments like heamatoporfirin and chlorophyll are used as photosensitizers. These materials are nowadays used for inhibiting cell growth, notably to treat such diseases as cancer, viral infections, intracellular parasitic infections, pulmonary fibrosis, hepatitis, liver cirrhosis, chronic nephritis, arteriosclerosis and other dysfunctions. It is a common practice for treatment of a health disorder of a recipient first to administer a suitable pigment photosensitizer to the recipient, then wait for a time span until a level of the photosensitizer rises up to an acceptable value in a target region conceived to be treated and, finally, to irradiate the target region with light, from a suitable light source. It is a disadvantage of the common practice that a limited selectivity is obtained, because the administered photosensitizers also reach secretion organs, like liver, presenting risks for these organs as well. Next to this, the source of light must be selected in accordance with a particular pigment which is selected as the photosensitizer, which corresponds to a narrow range of visible light spectrum and has a very small 1-2 mm penetration depth is tissue. Therefore, the common practice can provide acceptable results only for target regions that are not deep seated due to absorption of light in the tissue. It is a still further disadvantage of the common practice that the patient has to wait for 9 to 16 hours until the acceptable level of the photosensitizer has been built up in the target region.

An embodiment of the method as is set forth in the opening paragraph is known from EP1362598. The known method, addressing the drawbacks of the prior art, foresees means to substitute the light source by ultrasonic waves thereby selecting a class of photosensitizers that can be activated also by means of ultrasonic waves. The known method thereby considerably limits the scope of applicable materials which can be selected for treatment of deep seated targets.

It is a disadvantage of the known method that it requires additional hardware for implementing the activation of the photosensitizer, and is applicable to a limited range of known photosensitizers.

It is an object of the invention to provide a method for activating the photosensitizer which is easy to implement and is applicable for deep-seated targets.

To this end the method according to the invention comprises:
- selecting for the photosensitizer nanoparticles of a catalyst capable of catalyzing the production of an active oxygen form;
- irradiating the photosensitizer with light.

The technical measure of the invention is based on the insight that nanoparticles of a suitable catalyst capable of catalyzing the production of an active oxygen form, provide a plurality of advantageous effects when used for the photosensitizer. The term active oxygen refers to oxygen with an increased activation potential, for example (ε, Δ) singlet oxygen. Preferably, nanoparticles with dimensions of 0,5 - 200 nm are used, still preferably with dimensions of 0,5 - 100 nm, still preferably with dimensions of 0,5 - 50 nm. The nanoparticles may have spheric and/or rod shapes, their dimensions being measured by per se known Atomic Force Microscope (AFH) or Stonnich Tunnel (ST). It is noted that the photosensitizer according to the invention may comprise various mixtures of nanoparticle sizes, whereby also relative percentage of nanoparticles of specific dimensions may vary. The term dimension relates to all dimensions of nanoparticles.

First, it is found to be advantageous to use a metal-catalyst, notably those of a dioxide of a biologically compatible metal, as a photosensitizer, because it catalyzes a formation of active oxygen in response to an irradiation with light in a wide range of wavelengths, from ultra-violet to infra-red range. The photosensitizer is preferably irradiated beforehand, thereby, when the activated photosensitizer is administered to a recipient, only target region thereof is subjected to the action of the activated photosensitizer, sparing healthy regions. Additionally, the patient does not have to wait until the photosensitizer builds-up in the target region. Preferably, for the metal-catalyst dioxides of silver, iron and/or titanium are used. When the metal-catalyst is reduced in size down to particles of nanometer dimensions, it has an additional advantageous effect of the resulting matter being transparent to a wide range of wave lengths of light thereby improving an efficacy of the material response to light irradiation. In addition, such matter is capable of engaging in chemical reactions similarly to behavior of molecules constituting, for instance, a human being. Thus, from comparison between sizes of nanostructures and sizes of human cells, it follows that the nanostructures can be effectively transported even through the cell membranes, which improves their action on target region still further. Additionally, the nanostructures have a high surface of contact, thereby improving the efficacy of the activation process, which is substantially a mass exchange process dependent on the area of reacting surfaces. Moreover, the silver-, iron- and titanium-based metal-catalysts being a bio-compatible in nature produce no harmful effect to a biologic recipient.

The photosensitizer comprising nanoparticles of dioxide of a biologically compatible metal, being a substantially transparent medium, can be activated by visible light. Therefore such materials are perfectly suitable for use in a form of powder to be supplied locally on superficial targets. For example, a suitable amount of powder of nanoparticles can be applied to a target region and can be subjected to ambient light, or, alternatively, it can be subjected to another activation, for example by a laser, beforehand or during a suitable clinical or cosmetic procedure. It is noted that for superficial application active oxygen will be formed from the atmospheric oxygen and the action of the photosensitizer shall be as efficient.

In an embodiment of the method according to the invention the method further comprises adding the photosensitizer to a liquid yielding a suspension thereof and, preferably further adding oxygen to the thus formed suspension.

The technical measure of the invention is based on the insight that in order to prepare the activated photosensitizer for administering it into a recipient it is convenient to use liquid forms. Preferred embodiments of a liquid suitable to be used in the invention comprise water, solution of a colloid, liquid protein, for example albumin, or physiologic salt solution. Preferably, the resulting suspension is prepared using a ratio of at least 0,5 mg of a suitable metal dioxide per 1 liter of liquid. It is noted that when minerals are selected as a source of nanoparticles they generally comprise a mass fraction of 10% - 30% of respective metal dioxides. Therefore, for those minerals the total mass of prepared nanoparticles should be selected at least 5 mg per 1 liter of liquid. It is further noted that when nanoparticles are being used as photosensitizer, they act as catalysts for production of active oxygen forms, thereby not participating in chemical reactions. Therefore, the total amount of the photosensitizer is not a critical parameter for production of active oxygen, provided a minimum amount of 0,5 mg Ti02 or Fe02 or mixture thereof per liter of liquid is prepared. Because a surface interaction of the liquid with the ambient oxygen will not be sufficient to produce a necessary level of oxygenation of the suspension, the method according to the invention comprises the step of adding oxygen. Oxygen can be supplied from a suitable vessel my means of a pump. Preferable partial pressure of oxygen within the suspension is about 40 - 100 mmHg, still preferably about 70 mm Hg. Preferably, the photosensitizer is irradiated with a laser light with a wavelength in the range of 0,8 - 0,9 micrometer, which corresponds to a maximum absorption rate of oxygen and a region of optical transparency of tissues to laser light.

In a further embodiment of the method according to the invention, the method comprises activating the photosensitizer in a unit arranged to irradiate the suspension of the photosensitizer by light.

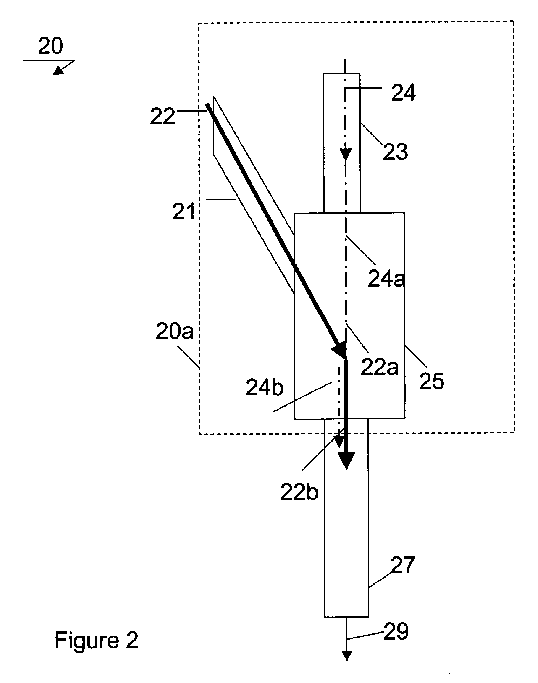

Preferably, a flow of the suspension comprising the photosensitizer is irradiated. By irradiating a flow of the suspension of the photosensitizer a production of active oxygen substantially in the whole volume of the photosensitizer is enabled. This step is advantageously carried out ex-vivo, thus outside the recipient, for example as a preparatory step for photodynamic treatment of a suitable health disorder. Preferably, light intensity of at least 1 Joule/ml of the suspension comprising the photosensitizer is used. Still preferably, the light intensity is selected in the range of 2 -3 Joules/ml of the suspension of the photosensitizer. It is further preferable that the flow of the suspension comprising the photosensitizer is selected in the range of 1 - 2 ml/second. Preferably, a suitable Y-shaped body is used for the unit, whereby a first arm of the Y-shaped body is used to supply a flow of the suspension of the photosensitizer into a main channel of the unit and a second arm of the Y-shaped body is used to supply light into the main channel of the Y-shaped body, preferably, by means of a suitably selected and attached optical fiber. Preferably, the flow rate of the suspension of the photosensitizer is selected in accordance with the light intensity supplied by the optical fiber. In general, a preferable relation between the flow rate and the light intensity lies in the range of 2-5 W of light energy per 1 ml of the suspension. Still preferably, the main channel of the Y-shaped body is attached to a storage cavity for storing the activated photosensitizer prior to its use. Preferably, the cavity is prepared in a sterile manner, so that the required volume of the activated photosensitizer can be extracted by, for example, an injection needle in a course of a medical treatment. A preferred embodiment of the unit will be described in more detail with reference to Figure 2. Alternatively, the unit can be arranged to conduct the activated photosensitizer to a supply unit which is used for administering the activated photosensitizer into the target region. In this case the flow of the photosensitizer advantageously serves as an optic conductor for the light waves, thereby further focusing a deposition of the active oxygen in the target region and conducting light to the tissue being treated. In case when laser light is selected for activation of the photosensitizer, its propagation in tissue causes local hyperthermia which can be seen as adjuvant real-time therapy still further improving medical efficacy of the method according to the invention.

In a still further embodiment of the method according to the invention the suspension is ozonated.

This procedure can be implemented using per se known apparata for ozonating liquids. The advantage of this step is in provision of an increased concentration of oxygen in the medium comprising the photosensitizer. Preferably, the level of ozonation is kept within a range of 5 - 10 mg ozon per 1 liter of the suspension comprising the photosensitizer. The process of ozonation is advantageous, because it provides a variety of active oxygen forms in the suspension, like the singlet oxygen and ozone, which complement each other in their chemical and biological activity.

In a still further embodiment of the invention for the photosensitizer nanoparticles of a heterocrystal mineral are selected. Preferable embodiments of the said mineral comprise rutile, sphene, loparite, perowskite, anatase, ilmenite, leukoxen, ferrite. By the term heterocrystal mineral one understands a substantially chemically homogenous substance, developed in a crystal within different lattices with respect to their types and shapes yielding polymorphic material which can be detected by a per se known DSC method.

Ferrites belong to a class of materials comprising dioxide of iron, most of them being ferromagnetic. Ferrites can refer to either a mineral or a chemically produced material using Fe203. Rutile (Ti02) is a natural mineral being a major ore of titanium, a metal used for high tech alloys because of its light weight, high strength and resistance to corrosion. Microscopic inclusions of rutile can be found in quartz, tourmaline, ruby, sapphire. Rutilated quartz can also be selected as a mineral source for production of nanoparticles. This stone is produced because at high temperatures and pressure, n(Si02)-n(Ti02) is in a stable state but as temperatures cool and pressure eases the two separate with rutile crystals trapped inside the quartz crystals. Minerals comprising sphene (Cao-Si02-TiO2), loparite (Ca, Ce, Na)(Nb, Ti))3, or (Ti, Nb)2(Na, Ca, Ce)206, perowskite (CaTi03), anatase (Ti02), or (Ti, Nb, Fe)02, ilmenite (FeTi03 and other modifications), leukoxen (TiO2Fe2O3+nH2O) are also suitable for production of photosensitizers in nanoparticle form. These materials have heterocrystal structure, wherein lattice fragments are coupled by means of hydroxide ion (OH-). Preferably, these materials are subjected to a thermal destruction for purposes of production of nanoparticles, whereby cooling is altered with heating. In course of the cooling phase, the chemical bonds of the hydroxide ion undergo crystallization thereby destructing the lattice. The action of the alternating cooling/heating mode can be seen as internal blasts causing the matter to reduce its size down to nanometers, thereby yielding nanoparticles. For this purpose a layer of a heterocrystal mineral is provided on a suitable carrier for enabling thermal contact with a suitable source, notably a thermo-element, coupled to a suitable power supply source. Preferably, a thickness of the layer is in the order of several millimeters, still preferably it is selected in the order of one millimeter, corresponding to a size of the lattice of rutile, sphene, loparite, perowskite, anatase, ilmenite, leukoxen and ferrite. The dimension of the layer in the longitudinal direction can be as large as one meter. Preferably, ceramics is selected for the carrier which can conduct energy in the range of 9 - 15 W/cm2. Preferably, the power supply source is controlled by a processor which is preferable operated by a suitable computer program arranged to control the duration of the cooling and heating modes. In accordance with the method of the invention, the power supply source produces pulses of constant current with amplitude of at least 10 A/mm2, which are transferred to a thermo-element. For elongated layers a plurality of thermo-elements can be used. The thermo-elements are conceived to operate in accordance with per se known thermoelectric effect of Peltier-Zeebeck, whereby upon application of a current of a suitable polarity the solder joints of the thermo-elements act as a cooler or as a heater. In the method according to the invention a pulse of a direct polarity is applied during a period of 10⁻⁴ to 1 second, as a result of which temperature of the surface of thermo-element facing the layer instantly decreases down to -50 degrees Celcius, preferably to -73 degrees Celsius. This causes the material of the layer to substantially cool, whereby the hydroxide groups of water molecules crystallize thereby induce miniature blasts in the material of the layer. After this, the pulse of the direct polarity is changed to a pulse of an inverse polarity, thereby causing the surface of thermo-elements facing the layer instantly to increase its temperature at least to + 80 degrees Celsius, preferably to +95 degrees Celsius, thereby melting water component in the crystal and disrupting lattice structure due to thermal vibration of lattice. After this, the polarity of the power supply unit is altered again, causing the crystallization of water component. Preferably, such alteration is applied during a period of 1 second to 1 minute. After a repetition of the cooling and heating modes, the layer is destructed down to nanoparticles. A number of the repetitions can be selected in accordance with desired dimension of nanoparticles. In general, in accordance with the method of the invention nanoparticles with dimensions of 0,5-200 nm can be produced.

The nanoparticles according to the invention exhibit high chemical activity, as active groups, initiating covalent and ionic bonds, are formed at parts of intermolecular breaks. Moreover, all dioxides of metals forming a basis of the compound still further increase their catalytic action for production of active oxygen. Under action of light photons, the oxygen then transforms into its singlet forms (ε, Δ), which oxidizing potential is substantially increased. Upon an interaction with other elements from environment the active oxygen and its secondary derivatives become a source of damage of a range of biologic objects which provide life supply to cells. As a result, in the biological matter, subjected to an interaction with the compound according to the invention, fibrous processes are initiated, as well as processes of blood and lymphatic blockage, acting as mechanical barrier for blood and lymphatic circulation, thereby isolating the tumor from supply sources. As a result the metabolic activity and invasive properties of tumor cells are substantially reduced and the tumor is thereby transferred into metabolically stable state.

In a still further embodiment of the method according to the invention the nanoparticles comprise a DNA-molecule.

Preferably, the nanoparticles are coupled to the DNA-molecule. It must be understood that the term "coupling" refers to either electrostatic coupling or to coupling due to covalent bonds. The DNA molecules undergo coupling after their mere addition to nanoparticles. Various embodiments of coupling are envisaged first, a single nanoparticle can be coupled to a single DNA molecule, secondly, one nanoparticle can be coupled to a plurality of DNA molecules, third, one DNA molecule can be coupled to a plurality of nanoparticles. Preferably, a sodium salt of DNA-molecules, commercially known as a "Derinat" is introduced into a suspension comprising the nanoparticles produced by means of destruction of a heterocrystal mineral, preferably based on a proportion of 1 portion of 1,5% solution of sodium salt per 1 portion of nanoparticles comprising at least 0,5 mg of suitable metal dioxide. This embodiment has a technical effect of still further increasing a selectivity of the nanoparticles in their biochemical action due to the fact that they are transported inside the cell by action of the DNA-molecule, where they act as the photosensitizer.

In a still further embodiment of the method according to the invention the nanoparticles comprise an anti-metabolic agent.

This technical measure is based on the insight that the nanometric compound can be used as a transport agent to transport a further medicament inside a cell. Preferably, the nanoparticles are coupled to the anti-metabolic agent, for example by means of electrostatic forces or covalent bonds. A coupling of the anti-metabolic agent to the nanoparticles may provide a cumulative cytotoxic action on a cell still further improving an efficacy of the thus prepared substance. For example, the anti-metabolic agent may comprise a per se known chemotherapeutic material.

A method for treatment of a health disorder of a recipient according to the invention comprises providing the recipient with activated photosensitizer in the form of nanoparticles, preferably nanoparticles of a heterocrystal mineral.

Preferably, the photosensitizer is activated by the method in accordance with any one of Claims 1-7, being set forth with reference to the foregoing. The method of treatment according to the invention is based on the insight that the thus prepared and irradiated photosensitizer exhibits superior properties in terms of biological effect and efficacy of a treatment procedure. The forming of the cytotoxic agent - the active oxygen, for instance in the form of singlet oxygen takes place ex-vivo after which the active medium is administered into the target region. This process reduces potential risks of the healthy tissue due to the fact that the active matter is transported locally and is not being absorbed by healthy tissues distant to the target region. Preferably, a light intensity of at least 1 Joule per 1 ml of liquid comprising photosensitizer is used. Several ways of administering the activated photosensitizer are envisaged.

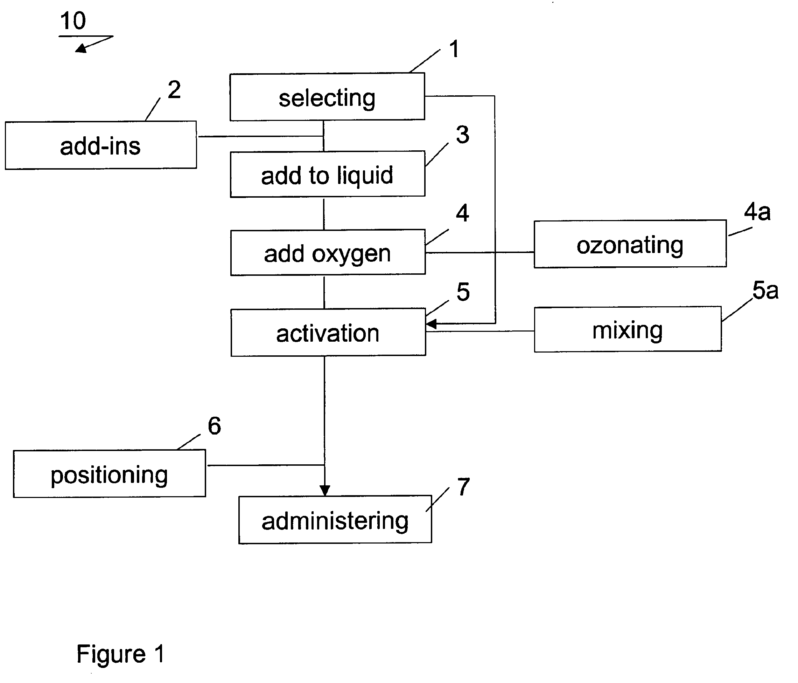

First, the activated photosensitizer is applicable for treatment of cancerous disease. In this case the prepared activated photosensitizer can be added to a liquid and be forwarded to an injection needle which can then be used to administer the thus formed suspension comprising active oxygen to the tumor. Preferably, the volume of the suspension to be injected in or close to the tumor is selected to be a factor of 5-10 greater than the volume of the tumor. The latter can be accessed a-priori or in real time using suitable medical imaging techniques known per se in the art. In case when the tumor is superficial, for example skin cancer, it is possible to use a suitable spraying device for pulverizing the activated suspension comprising the photosensitizer over the desired area. In case when a deeply seated tumor is treated, a required volume of the activated suspension comprising the photosensitizer is injected in the tumor or in vicinity thereof. The advantageous effects of the treatment of cancers will be further explained with reference to examples, discussed with reference to Figure 1. Preferably, the photosensitizer is coupled to a DNA-molecule. Still preferably, the photosensitizer is further coupled to an anti-metabolic agent for still further improving its cytotoxic action.

Secondly, it is possible to use the activated photosensitizer for treatment of wounds, ulcers, and for provision of a general antiseptic effect in treated area. For this purpose the activated photosensitizer can be administered by means of a spray, a powder application or the like to the area conceived to be treated. Use of photosensitizers per se for treatment of wounds is known from US 6, 107, 466. The known method the suitable photosensitizer is administered locally or systemically to the recipient, after which it is waited until the level of the photosensitizer has reached an effective tissue concentration at the wound site, after which a photo-activation is performed. For purposes of enabling the photo-activation, a pre-determined source of light is used, which is dependent on the type of photosensitizer being used.

It is further noted that in accordance with the foregoing, rutiles, sphenes, loparites, perowskites, anatases, ilmenites, leukoxenes and ferrites comprise silicon dioxide (Si02), which on its own has an advantageous effect of initiation tissue fibrosis. This advantageous effect, generally manifesting itself as a blood and lymphatic block, can be used for localization of cancerous tissue thereby for eliminating any source of nutrition of malignant cells. It is further noted that nanoparticles of Si02 can be used as transport agents, when chemically coupled to a suitable molecule. For example, nanoparticles of Si02 can be chemically coupled to colloid silver dioxide, which can supplement the action of Si02 by its photosensitizing properties. Alternatively, a Si02-coupled Ag02 can be added to nanoparticles comprising dioxide of titanium or dioxide of iron thereby complementing their action as metal catalysts for production of active oxygen forms.

The method according to the invention is superior over the method of wound treatment known from the prior art, particularly when nanoparticle photosensitizers based on bio-compatible metals are used, which are reduced in size to nanoparticles enabling activation of the photosensitizer using a broad range of available light sources, from ultra-violet to infra-read ranges for their activation. The method according to the invention is easy to implement and requires no latent phase for enabling an accumulation of the photosensitizer's concentration in the target area. Various means of deposition the activated photosensitizer in the wound are envisaged. It is possible to use a photosensitizer in the form of a powder. When an intra-operative treatment is envisaged, it is preferable to add the photosensitizer to a liquid thus yielding a suspension which can then be deposited on the target area using a spraying device, an injection needle, a tampon or a bandage material moistened with the activated suspension, or by means of a suitable catheter. The method according to the invention provided not only wound healing effect, but also shows an antiseptic action of the thus activated photosensitizer as well, due to generic anti-viral and anti-bacterial properties of the nanometric materials.

These and other aspects of the invention will be discussed with reference to figures.
Figure 1 presents in a schematic way an embodiment of a chart of the method according to the invention.
Figure 2 presents in a schematic way an embodiment of a unit for activating a flow of the photosensitizer.

Figure 1 presents in a schematic way an embodiment of a chart of the method 10 according to the invention. At step 1 of the method according to the invention the photosensitizer comprising in a form of, for example, nanoparticles of a dioxide of a biologically inert metal, for example titanium, silver or the like is selected. Preferably, the photosensitizer is provided in form of a powder. In case when the photosensitizer is envisaged to be used superficially it is activated at step 5 using a suitable source of light. It is noted that it is an advantage of nanometric materials than they can be activated by a wide range of light sources, including ambient light. Thus for treatment of a superficial disorder, like a wound, it may be sufficient to supply a suitable layer of the powder comprising the photosensitizer and activate it my means of sunlight. Also, in these conditions the powder will activate the ambient oxygen present in the atmosphere. Preferably, the powder is activated by means of an infra-red laser light with a wavelength in the range of 0,8 - 0,9 micrometer as this ranged of wavelengths corresponds to the maximum absorption rate of oxygen and to the region of optic transparency of tissue. Upon an event the photosensitizer is activated, it may be used at step 7 by administering it to a suitable target area of the recipient.

Alternatively, in case when a treatment of a deep seated target is envisaged, the method according to the invention proceeds to step 3 whereby the nanometric photosensitizer is added to a suitable liquid to yield a suspension. Suitable examples of the liquid comprise water, a solution of a colloid, liquid protein, like albumin, or the like, physiologic salt. Advantageously, prior to proceeding to step 3 of the method, substantial add-ins are added to the photosensitizer, for example DNA-molecules and anti-metabolic agents. When the photosensitizer engages in bonds with suitable portions of the DNA molecules and, optionally, with the anti-metabolic agent, the cytotoxic effect of the active oxygen catalyzed by the photosensitizer may be substantially increased.

At step 4 of the method according to the invention oxygen is provided to the suspension formed at step 3. Oxygen may be supplied from a suitable vessel by means of a pump. It is preferable to achieve a partial pressure of oxygen in the thus formed suspension of about 70 mm Hg. Still preferably, at step 4a a process of ozonation is performed for further increasing a concentration of oxygen in the vicinity of the photosensitizer. The process of ozonation may be performed using any per se known ozonating equipment. Due to ozonation also variety of active oxygen forms are provided.

Upon an event the liquid comprises a sufficient concentration of oxygen, the method according to the invention proceeds to step 5 whereby the active oxygen, notably a singlet oxygen, is produced by irradiating the liquid with a suitable source of light. As has been mentioned earlier, all light sources are suitable for this purpose, however, it is preferable to use an infra-red laser with the wavelength of 0,8 - 0,9 micrometer, which substantially corresponds to the maximum absorption rate of oxygen and a region of optical transparency of tissue. Still preferably, the activation is enabled using a mixing unit, which is discussed in further details with reference to Figure 2.

Upon an event the singlet oxygen is prepared, the liquid is ready to use, and may be stored, preferably in a sterile container. Alternatively, it may be transported by means of a suitable conductor to a treatment site whereby it can be administered to a recipient. In case when a target region of the recipient is deeply seated, the method according to the invention proceeds to step 6 whereby suitable medical diagnostic means are used to determine a spatial position of the target region. Preferably, ultra-sonic apparatus, is used. Alternatively, one may use an X-ray apparatus, a magnetic resonance imaging device, or a CT-scanner. Upon an event the position of the target region is determined, a suitable medical operator may provide the activated liquid into a suitable application unit, for example an injection needle, a catheter, or the like. Then, at step 7 of the invention, the activated liquid is administered to the recipient. The procedure and the clinical effect thereof will be discussed with reference to examples.

### Example 1.

For a superficially located cancerous mass the activated photosensitizer is introduced superficially and around the mass, for example, using a suitable injection needle. The volume of the suspension comprising the activated photosensitizer must be at least several times greater than the volume of the mass. Preferably it is 5 to 10 times greater than the volume of the mass. It is noted that it is possible to provide the injection needle with a Y-shaped body one arm being a supply channel for the suspension comprising the sensitizer, the other arm being an optical channel supplying the light. Preferably, the light intensity is not less than 1 W/cm2. Still preferably, the light is supplied in the direction of the flow of the suspension and is subjected to a further spreading in the tissue conceived to be treated. The flow rate of the liquid is preferably not less than 1 ml per 1 Joules of light energy. Preferably, to reduce pain sensation of the recipient a suitable anaestetic is introduced into the suspension prior to the procedure of intervention. Preferably, a 0.2% lidocaine and 10mg/l ozon are used.

### Case 1.

A patient B. aged 18, (dossier No. 212/01) was treated in the year 2000 for a recidive of a soft-tissue sarcoma in a right half of the chest. The recidive appeared after a time of three months of a tumor resection followed by a histological control thereof. During examination a spherical node of about 4 cm diameter was detected in a region of post-operative scar, said node being static, firmly attached to a rib. No distant metastases were detected by means of ultrasonic and X-ray investigation. The patient received interstitially 200 ml of the suspension comprising 1 mg of titanium dioxide prepared from rutile, irradiated by a laser light of 0,88 micrometer wavelength with an intensity of 5 W during 4 minutes. The procedure was repeated twice with a weekly interval. Tissue temperature in the course of treatment was not elevated by more than 0,8 degrees Celsius. A biopsy conducted three days after the second procedure demonstrated that the tumor belonged to sacroma's. After one year of follow-up no tumor growth was detected and in place of the recidive of 4cm in diameter one finds 1 cm node of fibrous tissue. During subsequent follow-up procedures no tumor manifestation was detected.

### Example 2

For deep seated tumors which cannot be localized by a mere inspection, diagnostic means are used for presenting data on the spatial location of the target region comprising the tumor and the dimensions thereof. Also in this embodiment, a suitably arranged injection needle can be used for depositing the activated photosensitizer in the tumor and/or around it.

### Case 2.

A patient K, aged 64 (dossier No. 4322) in 2001 was hospitalized in an urologic clinic due to disurrhea. In the course of examination adenocarcinoma of an upper portion of the left lobe of prostate gland was detected. Adjuvant to catheterization of the bladder, a treatment in according to the invention was carried out. Both lobes of the prostate gland received 250 ml of suspension comprising 1,5 mg dioxide of titanium in a solution of a colloid silver, irradiated by a laser light of 0.84 micrometer wavelength, intensity of 6 W, during 8 minutes. The tissue temperature was controlled and did not elevate by more than 1,2 degree Celsius in the course of treatment. The phenomenon of disurrhea has terminated and diagnostic screening showed solely a fibrous tissue in place of original tumor. This state was confirmed by a further follow-up three years after the treatment according to the invention.

### Case 3.

A female patient L., age 59 (dossier No. 176/32) was included in a group of volunteer patients to receive treatment in according to the invention in May 2004. The patient was diagnosed with a ductal type carcinoma of the left mamma, staged T3N2Mx. The radiotherapy received by the patient was not successful. During her investigation prior to treatment in accordance with the invention a tumor mass of substantial dimensions (10 cm3) was detected, which also had developed an ulcer. In the course of treatment the patient received 10 times intro-paratumoural injections with activated photosensitizer comprising 20% titanium dioxide, 30 % of calcium dioxide, and at least 15% of silicon dioxide. 5 mg of the photosensitizer was used to produce a suspension of about 1 liter in volume using sterile water, whereto 5 ml of 1.5% Derinat was added and 5 mg of the anti-metabolic agent in the form of doxorubicin. Next to the intra- and para-tumoral injections the lymph nodes received the suspension of the activated photosensitizer as well. During a course of the follow-up in November 2004 no manifestation of cancerous cells in the treated volume was detected, which was confirmed by substantial hystologic analysis. The area of the ulcer defect was reduced by about 5 times and no signs of any inflammatory process can be seen as well. In March 2005 dermal defect of ulcers was closed by plastic surgery.

### Example 3.

Adjuvant wound healing effect, for nanoparticles of photosensitizer comprising a stimulator of fibrosis.

### Case 4.

A female patient 76 years (case No. 318/A), after being a patient of a plurality of oncology clinics during a period of more than four years due to presence of a substantial (18 cm3) cancerous ulcer on her left check. Conventional attempts to close the dermal defect were not successful. Then she was subjected to a monthly course of treatment with nanoparticles prepared using a mixture of 10 mg ferrite and 10 mg Si02, whereby the nanoparticles were administered in form of dry powder. Upon the application of the powder on the ulcer, it was irradiated with a halogen lamp with intensity of 3 W/cm2 in dose of 1,3 kJoule. After the treatment the skin defect disappeared, whereby the prints of ulcer showed forms of fibrous cells in phases of apoptosis. It is noted that although the present example shows a cumulative action of nanoparticles of iron dioxide and silicon dioxide, the former was used to treat cancer and the latter was used as an adjuvant therapeutic agent for stimulating fibrosis to close the skin defect. It must be understood, that clinical use of nanoparticles prepared from quartzite for wound healing is contemplated hereby as such.

Figure 2 presents in a schematic way an embodiment 20 of a unit for activating the photosensitizer, notably a flowing photosensitizer. The unit 20a comprises a Y-shaped body, one arm 21 being conceived to receive a flowing photosensitizer 21, the other arm 23 being conceived to supply light waves 24 from a light source (not shown), preferably using a suitable optical fiber. The Y-shaped body of the unit 20a is arranged in such a way that the flow of the liquid photosensitizer 22a is fully irradiated by the light beam 24a. This arrangement ensures complete activation of the photosensitizer in the whole irradiated volume. The portion 25 of the Y-shaped body may be manufactured with considerable dimensions, to contain the whole volume of the photosensitizer conceived to be used during a treatment procedure. It is found to be sufficient to provide the portion 25 with a 10 - 500 ml volume. Preferably, the volume of the portion 25 is selected around 50 ml. When the photosensitizer is activated and is stored in the compartment 25, a supply unit 27 can be connected to its distal portion. The supply unit may comprise a catheter, an injection needle, a spraying device used for administering the photosensitizer 29 to the target region of the recipient. In case when the injection needle, or a catheter are used is may be preferable not to interrupt the flow 22b of the activated photosensitizer allowing it to conduct the optical radiation into the tissues of the recipient. In this way the administration of the activated photosensitizer is performed substantially at the same time with its activation, whereby the liquid photosensitizer acts as an optical conductor of the light beam into the tissue. This procedure has an advantageous clinical effect as the nanometric photosensitizer instantly deposits the dose of the activated oxygen and the optical dose at the micro-level as well. In case when the laser light is used the optical dose thus delivered causes additional tissue damage due to local ablation due to laser-induced hyperthermia. Preferably, the laser light with the wavelength of 0,8 - 0,9 micrometer is used, corresponding to the maximum absorption rate of oxygen and the region of optical transparency of tissue.

## Claims

1. A method of activating a photosensitizer, comprising :
- selecting for the photosensitizer nanoparticles comprising a catalyst capable of catalyzing the production of an active oxygen form;
- irradiating the nanoparticles with light.

2. A method according to Claim 1, wherein the method comprises:
- adding the photosensitizer to a liquid yielding a suspension thereof;
- adding oxygen to the thus formed suspension.

3. A method according to Claim 2, wherein the step of irradiating the photosensitizer is performed in a unit arranged to irradiate the suspension of the photosensitizer by said light.

4. A method according to Claims 2 or 3, comprising the step of ozonating the suspension.

5. A method according to any one of the preceding Claims, wherein the nanoparticles are nanoparticles of a heterocrystal mineral.

6. A method according to any one of the preceding Claims 1 - 5, wherein the nanoparticles comprise a DNA-molecule.

7. A method according to any one of the preceding Claims 1 - 6, wherein the nanoparticles comprise an anti-metabolic agent.

8. A method for treating a health disorder of a recipient, comprising providing the recipient activated photosensitizer in the form of nanoparticles, preferably nanoparticles of a heterocrystal mineral.

9. A method according to Claim 8, wherein the photosensitizer has been activated by a method in accordance with any one of the preceding Claims 1-7.

10. A method according to any one of the preceding Claims 8 - 9, wherein the photosensitizer is administered by means of an application unit in a target region of the recipient, the method further comprising:
- determining a spatial position of the target region;
- positioning the application unit in accordance with the determined spatial position.
